Europäisches Patentamt

European Patent Office

Office européen des brevets

(11) Publication number: **0 442 185 A1**

# EUROPEAN PATENT APPLICATION

(21) Application number: 90301677.2

(22) Date of filing: 16.02.90

(51) Int. Cl.⁵: **C08K 3/16**, D06M 11/17, D06M 11/45, D06M 15/19, B32B 27/12, C08J 5/10

(43) Date of publication of application:
21.08.91 Bulletin 91/34

(84) Designated Contracting States:
BE CH DE ES FR GB IT LI NL SE

(71) Applicant: NIPPON SHOKUBAI KAGAKU KOGYO CO. LTD.
1-1, Koraibashi, 4-chome
Chuo-ku Osaka-shi Osaka-fu(JP)

(72) Inventor: Harada, Nobuyuki
41-3-1007, 4-chome
Yamadahigashi, Suita-shi, Osaka-fu(JP)
Inventor: Motono, Yoshihiro
931-11 Hamada
Aboshi-ku, Himeji-shi, Hyogo-ken(JP)
Inventor: Shimomura, Tadao
9-11, 2-chome, Shinsenrinishimachi
Toyonaka-shi, Osaka-fu(JP)

(74) Representative: Rees, David Christopher et al
Kilburn & Strode 30 John Street
London WC1N 2DD(GB)

(54) Absorbent composite and method for production thereof.

(57) An absorbent composite comprising 100 parts by weight of an absorbent polymer, 1 to 600 parts by weight of a fibrous substrate, and 0.1 to 50 parts by weight of polyaluminium chloride and having said absorbent polymer adjoined to and fixed on said fibrous substrate through the medium of said polyaluminum chloride as a binder,

EP 0 442 185 A1

This invention relates to an absorbent composite and a method for the production thereof. More particularly, it relates to an absorbent composite which has an absorbent polymer firmly fixed on a fibrous substrate.

In recent years, absorbent polymers capable of absorbing water in amounts some ten to some hundred times their own weights have been developed and have been finding extensive utility in agricultural, forestry, civil engineering and sanitary fields, for example disposable diapers and sanitary napkins.

These absorbent polymers are generally in a powdery form. Since they do not exhibit a sufficient rate of absorption when they are used by themselves, conversion into composites by their combination with various fibrous substrates has been proposed [Japanese Patent Laid-Open SHO 56(1981)-129,165, Japanese Patent Laid-Open SHO 55(1980)-148,154, Japanese Patent Laid-Open SHO 56(1981)-2,163, Japanese Patent Laid-Open SHO 58(1983)-131,133, Japanese Patent Laid-Open SHO 58(1983)-84,804, Japanese Patent Laid-Open SHO 59(1984)-86,657, Japanese Patent Laid-Open SHO 61(1986)-62,463, and Japanese Patent Laid-Open SHO 63(1988)-63,723].

The conversion of these absorbent polymers into the composites by simply depositing the absorbent polymers on fibrous substrates is effective in improving the absorbent polymers' rate of absorption. However, the composites produced, under the conditions of their actual use, are deficient in their capacity and absorption rate under the application of pressure. In addition, absorbent composites produced by the conventional methods of depositing absorbent polymers on fibrous substrates have a disadvantage that the hydrated gels of absorbent polymers resulting from the absorption of an aqueous liquid are readily separable from the fibrous substrates.

An object of this invention, therefore, is to provide an absorbent composite having an absorbent polymer fixed firmly on a fibrous substrate, which exhibits a notably improved capacity for absorption under the application of pressure, and a method for the production of the absorbent composite.

Another object of this invention is to provide an absorbent composite which has an absorbent polymer fixed so firmly on a fibrous substrate that even after the absorbent polymer is converted into a hydrated gel in consequence of absorption of an aqueous liquid, the absorbent polymer now in the form of the hydrated gel remains inseparably on the fibrous substrate, and a method for the production of the absorbent composite.

According to the present invention, there is provided an absorbent composite comprising 100 parts by weight of an absorbent polymer, 1 to 600 parts by weight of a fibrous substrate, and 0.1 to 50 parts by weight of polyaluminium chloride and having the absorbent polymer adjoined to and fixed on the fibrous substrate through the medium of the polyaluminium chloride as a binder.

These objects are also accomplished by a method for the production of an absorbent composite, which comprises causing 100 parts by weight of an absorbent polymer to contact 1 to 600 parts by weight of a fibrous substrate in the presence of an aqueous liquid containing 0.1 to 50 parts by weight of polyaluminium chloride.

This invention has originated in the new knowledge that polyaluminium chloride is particularly useful as a binder for fixing an absorbent polymer on a fibrous substrate. In accordance with this invention, an absorbent composite possessing previously unattainable excellent absorption characteristics and exhibiting the ability to retain its shape even after absorption of an aqueous liquid may be obtained by uniting polyaluminium chloride and a fibrous substrate with an absorbent polymer in a specific quantitative ratio in the presence of an aqueous liquid.

The absorbent composite of this invention, owing to the synergistic effect produced by the absorbent polymer, the polyaluminium chloride, and the fibrous substrate in the united mass, enjoys a considerable improvement in its capacity and absorption rate under pressure. Thus, it possesses previously unattainable excellent absorption characteristics.

The absorbent composite of this invention, therefore can be combined with other materials capable of absorbing, diffusing, and retaining an aqueous liquid and, in the combined state, may be used advantageously as absorbents in sanitary articles such as sanitary napkins and disposable diapers. It may also be advantageously used in various applications necessitating the retention and absorption of water such as are found in medical, agricultural and horticultural fields, civil engineering fields, food processing fields, and other industrial fields.

The term "absorbent polymer" as used in the present invention refers to a polymer of the type which, on exposure to water, swells and forms a hydrated gel (hydrogel) by absorbing a large amount of water. The absorbent polymers which answer this description include a hydrolyzed starch-acrylonitrile graft copolymer, a neutralized starch-acrylic acid graft copolymer, a saponified acrylic ester-vinyl acetate copolymer, a hydrolyzed acrylonitrile copolymer or acrylamide copolymer, a modified cross-linked polyvinyl alcohol, a neutralized self-cross-linking polyacrylic acid, a cross-linked polyacrylate salt, and a neutralized

2

cross-linked isobutylene-maleic anhydride copolymer, for example. Since these absorbent polymers generally form a hydrogel, they possess a cross-linked structure in their polymers. For this invention, the absorbent polymers possessing a uniform cross linking density and those having a cross linking density heightened in the surface region by a surface treatment are both usable.

In these absorbent polymers, cross-linked polymers of water-soluble ethylenically unsaturated monomers having acrylic acid (or a salt thereof) as a main component thereof prove to be particularly desirable. In these cross-linked polymers, cross-linked copolymers of acrylic acid with acrylate salt (molar ratio in the range of 5 : 95 to 50 : 50) and partially neutralized cross-linked polyacrylic acid (neutralization ratio in the range of 50 to 95 mol%) are particularly desirable. The salt as comonomers in the copolymers mentioned above is desired to be a salt of such alkali metals as sodium and potassium and ammonium salt.

The form of such an absorbent polymer is not critical. When the absorbent polymer is in the form of powder, granules, fibres or sheet, for example, it can be used safely for the production of the composite contemplated by this invention.

The polyaluminium chloride to be used in this invention is the product of polymerization of basic aluminum chloride and is represented by the general formula, $[Al_2(OH)_n Cl_{6-n}]_m$, wherein n satisfies $1 < n < 5$ and m satisfies $m \leq 10$.

The amount of this polyaluminium chloride to be used in the absorbent composite is in the range of 0.1 to 50 parts by weight, preferably 1 to 30 parts by weight, based on 100 parts by weight of the absorbent polymer. If this amount is less than 0.1 part by weight, an absorbent composite having an absorbent polymer fixed firmly on a fibrous substrate is not obtained. If this amount exceeds 50 parts by weight, the amount of the adsorbent polymer to be incorporated in the absorbent composite proportionately decreases and the saturated absorption capacity of the produced absorbent composite is not sufficient.

The fibrous substrates which are usable in this invention include synthetic fibres of polyamide, polyacrylonitrile, polyester, and polyurethane and cellulose fibres of cotton, rayon, and pulp, for example. These fibres are used in the form of long-staple fibres or short-staple fibres or in the form of sheets such as paper, non-woven fabric, woven fabric, and knitted fabric which are obtained by moulding or interlacing such fibres.

The amount of the fibrous substrate to be incorporated in the absorbent composite is in the range of 1 to 600 parts by weight, preferably 2 to 300 parts by weight, based on 100 parts by weight of the absorbent polymer. If this amount is less than 1 part by weight, the produced absorbent composite is deficient in its absorption rate. If the amount exceeds 600 parts by weight, the absorption capacity exhibited by the produced absorbent composite under application of pressure is unduly low.

The absorbent composite of this invention is obtained by mutual adhesion of the absorbent polymer and the fibrous substrate through the medium of the polyaluminum chloride as a binder component. This mutual adhesion is accomplished by causing the absorbent polymer and the fibrous substrate to contact the polyaluminium chloride in a prescribed ratio in the presence of an aqueous liquid. This procedure, when necessary, may further comprise a step of drying or compression moulding.

The specific methods by which the production of the absorbent composite of this invention is attained include those indicated below, for example.

(1) A method which comprises mixing the absorbent polymer with a solution containing the polyaluminium chloride, spreading or spraying the resultant mixed solution on the fibrous substrate, and thereafter drying the resultant composite desirably at a temperature in the range of 60 to 180°C.

(2) A method which comprises immersing the fibrous substrate in the mixed solution prepared in the method of (1), then removing the substrate from the mixed solution, and drying the wet substrate.

(3) A method which comprises mixing the fibrous substrate and the absorbent polymer with a solution containing the polyaluminium chloride, moulding the resultant mixture in the form of sheet, and drying the resultant sheet.

(4) A method which comprises dry mixing the fibrous substrate with the absorbent polymer, adding a solution containing the polyaluminium chloride to the resultant mixture, and, if necessary, drying the resultant composite.

(5) A method which comprises dry mixing the fibrous substrate, the absorbent polymer, and the polyaluminium chloride, adding an aqueous liquid or steam to the resultant mixture, and, if necessary, drying the resultant composite.

(6) A method which comprises mixing the absorbent polymer thoroughly swelled in advance with an aqueous liquid with the polyaluminium chloride and the fibrous substrate, and drying the resultant composite.

(7) A method which comprises dry mixing the absrobent polymer and the polyaluminium chloride, mixing the resultant mixture with the fibrous substrate wetted in advance with an aqueous liquid, and optionally drying the resultant composite.

(8) A method which comprises mixing the absorbent polymer with the fibrous substrate wetted in advance with an aqueous solution of the polyaluminium chloride and optionally drying the resultant mixture.

Optionally, during the drying of the produced composite in these methods, a press may be used in order for the composite to be simultaneously dried and compression moulded.

The aqueous liquid to be used in this invention has no particular restriction except for the sole requirement that it should be capable of dissolving the polyaluminium chloride. The aqueous liquids which are usable herein include water and mixed solutions of water with hydrophilic organic solvents such as methanol and ethanol, for example. Optionally, the aqueous liquid may incorporate therein deodorant, antioxidant, antifungal agent, fungicide, herbicide, fertiliser, perfume, ultraviolet absorbent, etc. to suit the purpose for which the absorbent composite is used. Further, the aqueous liquid may contain a thickening agent such as hydroxyethyl cellulose and methyl cellulose in order to improve the coat-workability.

Now, the present invention will be described more specifically below with reference to working examples. It should be noted, however, that this invention is not limited in any respect to these examples. The magnitudes of the capacity for absorption, the amount of absorption under application of pressure, and the ratio of separation of absorbent polymer mentioned hereinafter are those determined respectively by the methods indicated below.

(1) Capacity of absorbent composite for absorption

This property was determined by keeping about 0.2 g ($W_o$) of a sample absorbent composite stirred and immersed in 200 g of physiological saline solution for 30 minutes, then collecting the swelled absorbent composite on tissue paper, draining them both, and checking the weight (W) of the drained absorbent composite.

The capacity of the absorbent composite for absorption was calculated in accordance with the following formula using the found weight (W).

Capacity for absorption (g/g) = $W/W_o$

wherein $W_o$ is the weight of the absorbent composite before the absorption and W is the weight of the absorbent composite after the absorption.

(2) Ratio of separation of absorbent polymer

This property was determined by removing the swelled absorbent composite obtained in the procedure of (1), filtering the residual physiological saline solution thereby collecting the hydrated gel of the absorbed polymer separated from the fibrous substrate and dispersed in the physiological saline solution, and taking the dry weight of this gel.

This ratio of separation of the absorbent polymer was calculated in accordance with the following formula using the found weight.

$$\text{Ratio of separation of absorbent polymer (\% by weight)} = \frac{\text{Weight of separated polymer (g)}}{\text{Weight of polymer adhering to fibrous substrate (g)}} \times 100$$

(3) Amount of absorption under application of pressure

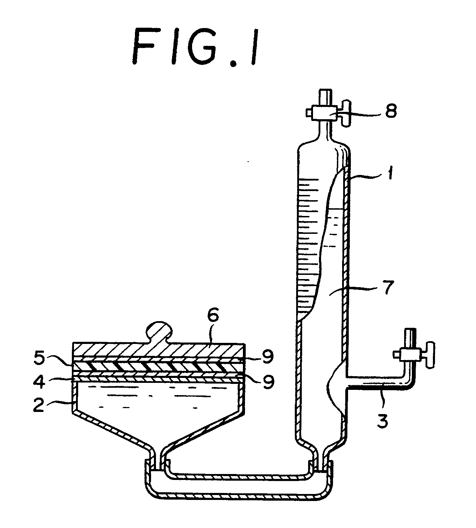

This property was determined by the use of the device shown schematically in Figure 1. This determination was performed by first placing artificial urine (aqueous solution containing 1.9% by weight of urea, 0.8% by weight of sodium chloride, 0.1% by weight of calcium chloride, and 0.1% by weight of magnesium sulphate) 7 in a buret 1, closing a cock 8 in the upper inlet of the buret, setting a measuring base 2 on the same level as a closed air inlet 3 to the buret, opening the air inlet 3 of the buret 1, mounting 0.3 g of a sample absorbent composite 5 interposed between two opposed circular sheets of filter paper (Toyo Filter Paper No. 2) 9 measuring 9 cm in diameter on a glass filter 4 measuring 90 mm in diameter laid on the measuring base 2, exerting a load 6 (20 g/cm²) measuring 9 cm in diameter on the sample for 10 minutes, finding the amount (W) of the artificial urine absorbed by the filter paper and the absorbent composite from the scale reading of the buret, and separately finding as a blank ($W_0$) the amount of urine similarly absorbed without use of the absorbent composite. The amount of absorption was calculated in accordance with the following formula using the found amounts.

$$\text{Amount of absorption under application of pressure (ml/g)} = \frac{[\text{Amount of artificial urine absorbed (W)}] - [\text{Blank amount of absorption } (W_0)]}{\text{Weight of absorbent composite}}$$

Reference Example 1

In an atmosphere of nitrogen gas, 4,000 parts by weight of an aqueous 40% solution of an acrylate type monomer containing 74.98 mol% of sodium acrylate, 25 mol% of acrylic acid, and 0.02 mol% of trimethylol propane triacrylate was subjected to stationary polymerization using 0.5 part by weight of ammonium persulphate and 0.1 part by weight of sodium hydrogen sulphate at a temperature in the range of 50° to 80°C to obtain a gel-like hydrated polymer. This gel-like hydrated polymer was dried in a hot air drier at 180°C, pulverized with a hammer type pulverizer, and sifted with a 20-mesh metallic gauze to obtain a 20-mesh pass powder (hereinafter referred to as "Absorbent Polymer (1)").

Reference Example 2

In a reaction vessel, 0.7 part by weight of sorbitan monostearate was dissolved in 300 parts by weight of n-hexane. Separately, an aqueous monomer solution was prepared by dissolving 30 parts by weight of acrylic acid and 0.006 part by weight of methylene bisacrylamide in 63 parts by weight of water, neutralizing the resultant solution by the addition of 12.5 parts by weight of sodium hydroxide, and further dissolving 0.05 part by weight of potassium persulphate in the neutralized solution.

Then, the aqueous monomer solution was added to and suspended in the solution prepared as described above in the reaction vessel and the resultant suspension was polymerized under a current of nitrogen gas at 65°C for 5 hours. After the polymerization was completed, the polymerization product was dried under a vacuum to produce a powder (hereinafter referred to as "Absorbent Polymer (2)").

Example 1

In a liquid obtained by mixing 100 parts by weight of Absorbent Polymer (1) with 10 parts by weight of polyaluminium chloride, (manufactured by Kishida Kagaku K.K.), 600 parts of methanol, and 1,000 parts by weight of water, 60 parts by weight of a polyester nonwoven fabric having a basis weight of 32 g/m² was immersed. The wet non-woven fabric was pulled out on a metallic gauze and left draining thereon. The drained fabric was dried in a hot air drier at 120°C for 10 minutes to obtain an Absorbent Composite (1).

The absorbent composite (1) thus obtained was found to have a basis weight of 80 g/m².

Example 2

A liquid obtained by mixing 200 parts by weight of Absorbent Polymer (2) with 20 parts by weight of polyaluminium chloride, 600 parts by weight of methanol, and 1,000 parts by weight of water was applied to a polyester non-woven fabric having a basis weight of 32 g/m². The resultant coated fabric was dried in a hot air drier at 120°C for 15 minutes, to obtain an Absorbent Composite (2).

The absorbent composite (2) thus obtained was found to have a basis weight of 130 g/m².

Example 3

In a liquid obtained by mixing 100 parts by weight of Absorbent Polymer (2) with 20 parts by weight of polyaluminium chloride, 600 parts by weight of ethanol, and 1,000 parts by weight of water, 80 parts by weight of a wood pulp fibre sheet having a basis weight of 40 g/m² and a thickness of 1 mm was immersed.

5

The wet sheet was pulled out on a metallic gauze and left draining thereon. The drained sheet was vacuum dried at 80°C to obtain an Absorbent Composite (3).

The absorbent composite (3) thus obtained was found to have a basis weight of 90 g/m².

Example 4

An Absorbent Composite (4) was obtained by mixing 100 parts of fluff pulp with 1,000 parts by weight of an aqueous 2 wt% polyaluminium chloride solution thereby preparing a pulp slurry, mixing this pulp slurry with 100 parts by weight of Absorbent Polymer (2), and then vacuum drying the slurry at 120°C.

Example 5

An Absorbent Composite (5) was obtained by mixing 100 parts by weight of an aqueous 2 wt% pulp slurry prepared in advance by disintegrating virgin pulp in water with 150 parts by weight of ethanol, 0.25 part by weight of polyaluminium chloride, and 1.0 part by weight of Absorbent Polymer (2), moulding the resultant mixed solution in the form of a sheet on a 200-mesh metallic gauze, and drying the resultant sheet at 120°C for 5 minutes.

Examples 6 to 9

Absorbent Composites (6), (7), (8), and (9) were obtained by following the procedure of Example 1, except that the amount of polyaluminium chloride to be used was changed respectively to 30, 20, 5, and 1 part by weight. The physical properties of the absorbent composites were as shown in Table 1.

The absorbent composites (6), (7), (8), and (9) invariably had a basis weight of 80 g/m².

Control 1

An Absorbent Composite (1) for Comparison was obtained by following the procedure of Example 1, except that the use of polyaluminium chloride was omitted.

The absorbent composite (1) for comparison was found to have a basis weight of 60 g/m².

Control 2

An Absorbent Composite (2) for comparison was obtained by following the procedure of Example 9, except that 1 part by weight of aluminium chloride was used in the place of 1 part by weight of polyaluminium chloride.

The absorbent composite (2) for comparison was found to have a basis weight of 62 g/m².

Control 3

An Absorbent Composite (3) for comparison was obtained by following the procedure of Example 8, except that 5 parts by weight of polyoxyethylene glycidyl ether (produced by Nagase Kasei Kogyo K.K. and marketed under trademark designation of "Denacol EX-810") was used in the place of 5 parts by weight of polyaluminium chloride.

The absorbent composite (3) for comparison thus obtained was found to have a basis weight of 65 g/m².

Example 10

An Absorbent Composite (10) was obtained by mixing 100 parts by weight of the absorbent polymer (2) with 20 parts by weight of polyaluminium chloride, 600 parts by weight of methanol, 1,000 parts by weight of water, and 8 parts by weight of methyl cellulose (produced by Shin-etsu Chemical Industry Co., Ltd. and marketed under trademark designation of "Metholose 60SH") thereby preparing a liquid, applying the liquid to 60 parts of a polyester non-woven fabric having a basis weight of 32 g/m², and then drying the coated fabric in a hot air drier at 120°C for 15 minutes.

The absorbent composite (10) thus obtained was found to have a basis weight of 82 g/m².

The absorbent composites (1) to (10) and the absorbent composites (1) to (3) for comparison obtained respectively in Examples 1 to 10 and in Controls 1 to 3 were tested for capacity for absorption, amount of

EP 0 442 185 A1

absorption under pressure, and ratio of separation of absorbent polymer by the methods described above. The results were as shown in Table 1.

Table 1

| | Capacity of absorbent composite for absorption (g/g) | Ratio of separation of absorbent polymer (%) | Amount of absorption under pressure (ml/g) |
|---|---|---|---|
| Absorbent composite (1) | 30 | 1 | 29 |
| Absorbent composite (2) | 35 | 2 | 33 |
| Absorbent composite (3) | 25 | 1 | 24 |
| Absorbent composite (4) | 27 | 4 | 25 |
| Absorbent composite (5) | 20 | 0.7 | 18 |
| Absorbent composite (6) | 23 | 0.5 | 22 |
| Absorbent composite (7) | 29 | 0.8 | 25 |
| Absorbent composite (8) | 35 | 1.3 | 28 |
| Absorbent composite (9) | 40 | 3 | 30 |
| Absorbent composite (10) | 29 | 1.1 | 27 |
| Absorbent composite for comparison (1) | – | 100 | 10 |
| Absorbent composite for comparison (2) | – | 100 | 13 |
| Absorbent composite for comparison (3) | 15 | 34 | 14 |

## Claims

1. An absorbent composite comprising 100 parts by weight of an absorbent polymer and 1 to 600 parts by weight of a fibrous substrate, characterised by further comprising 0.1 to 50 parts by weight of polyaluminium chloride, the absorbent polymer being adjoined to and fixed on the fibrous substrate through the medium of the polyaluminium chloride as a binder.

2. An absorbent composite as claimed in Claim 1, characterised in that the absorbent polymer is a cross-linked polymer of a water-soluble ethylenically unsaturated monomer, having acrylic acid or a salt thereof as a main component, such as a cross-linked copolymer of acrylic acid with an acrylate salt, with a molar ratio in the range of 5 : 95 to 50 : 50 or a partially neutralised cross-linked polyacrylic acid with a neutralisation ratio in the range of 50 to 95 mol %.

3. An absorbent composite as claimed in Claim 1 or Claim 2, characterised in that the amount of the fibrous substrate is in the range of 2 to 300 parts by weight and that of the polyaluminium chloride in the range of 1 to 30 parts by weight, respectively based on 100 parts by weight of the absorbent polymer.

7

4. An absorbent composite as claimed in any preceding claim, characterised in that the fibrous substrate is a sheetlike article such as paper, nonwoven fabric, woven fabric, and/or knitted fabric.

5. A method for the production of an absorbent composite as defined in any preceding claim, the method comprising causing the absorbent polymer to contact the fibrous substrate in the presence of an aqueous liquid containing the 0.1 to 50 parts by weight of the polyaluminium chloride.

6. A method as claimed in Claim 5, characterised in that the aqueous liquid is water or a mixture of water and a hydrophilic organic solvent.

7. A method as claimed in Claim 5 or Claim 6, characterised by mixing the absorbent polymer with a solution of the polyaluminium chloride, causing the resultant mixture to contact the fibrous substrate, and drying the resultant wet substrate.

8. A method as claimed in Claim 5 or Claim 6, characterised by mixing the fibrous substrate and the absorbent polymer with a solution of the polyaluminium chloride, moulding the resultant mixture in the form of sheet and drying the resultant sheet.

9. A method as claimed in Claim 5 or Claim 6, characterised by dry mixing the fibrous substrate with the absorbent polymer, and adding a solution of the polyaluminium chloride to the resultant mixture.

10. A method as claimed in Claim 5 or Claim 6, characterised by dry mixing the fibrous substrate with the absorbent polymer and the polyaluminium chloride, and adding an aqueous liquid or steam to the resultant mixture.

11. A method as claimed in Claim 5 or Claim 6, characterised by thoroughly swelling the absorbent polymer with an aqueous liquid, mixing the resultant swelled absorbent polymer with the polyaluminium chloride and the fibrous substrate, and drying the resultant mixture.

12. A method as claimed in Claim 5 or Claim 6, characterised by dry mixing the absorbent polymer with the polyaluminium chloride and mixing the resultant mixture with the fibrous substrate wetted in advance with an aqueous liquid.

13. A method as claimed in Claim 5 or Claim 6, characterised by wetting the fibrous substrate with a solution of the polyaluminium chloride and mixing the resultant wetted fibrous substrate with the absorbent polymer.

# FIG.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4888238 (KATZ ET AL) <br> * column 1, lines 51 - 62; claims 1-3, 7, 24, 26 * | 1, 5-7 | C08K3/16 <br> D06M11/17 <br> D06M11/45 <br> D06M15/19 |
| A | EP-A-233067 (SEKISUI PLASTICS CO LTD) <br> * claim 1 * | 1 | B32B27/12 <br> C08J5/10 |
| A | US-A-4558091 (HUBBARD) <br> * column 3, line 59 - column 4, line 68 * | 1, 5, 12 | |

TECHNICAL FIELDS
SEARCHED (Int. Cl.5)

C08K
C08F

The present search report has been drawn up for all claims

| Place of search | Date of completion of the search | Examiner |
|---|---|---|
| THE HAGUE | 09 OCTOBER 1990 | SCHUELER D.H.H |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)